# EUROPÄISCHE PATENTSCHRIFT

(11) **EP 1 217 998 B1**
(45) Veröffentlichungstag und Bekanntmachung des Hinweises auf die Patenterteilung: **01.08.2007**
(21) Anmeldenummer: 00969311.0
(22) Anmeldetag: 27.09.2000
(51) Int. Cl.: A61K 31/137, A61P 13/10

(54) **VERWENDUNG VON (+)-TRAMADOL, O-DEMETHYLTRAMADOL, O-DESMETHYL-N-MONO-DESMETHYL-TRAMADOL ZUR THERAPIE DER HARNINKONTINENZ**
USE OF (+)-TRAMADOL, O-DEMETHYLTRAMADOL, O-DESMETHYL-N-MONO-DESMETHYL-TRAMADOL FOR TREATING URINARY INCONTINENCE
UTILISATION DE (+)-TRAMADOL, DE O-DEMETHYLTRAMADOL, DE O-DESMETHYLE-N-MONO-DESMETHYL-TRAMADOL POUR TRAITER L'INCONTINENCE URINAIRE

(30) Priorität: 05.10.1999 DE 19947747; 21.02.2000 DE 20002943 U
(43) Veröffentlichungstag der Anmeldung: 03.07.2002
(73) Patentinhaber: Grünenthal GmbH, 52078 Aachen (DE)
(72) Erfinder: CHRISTOPH, Thomas, 52080 Aachen (DE); FRIDERICHS, Elmar, 52223 Stolberg (DE)
(86) Internationale Anmeldenummer: PCT/EP2000/009420
(87) Internationale Veröffentlichungsnummer: WO 2001/024783

(56) Entgegenhaltungen:
- EP-A- 0 693 475
- EP-A- 0 870 499
- WO-A-98/40053
- WO-A-98/46216
- WO-A-98/52929
- RAFFA ET AL.: JOURNAL OF PHARMACOLOGY AND EXPERIMENTAL THERAPEUTICS, 1993, Seiten 331-340,
- GILLEN ET AL.: NAUNYN-SCHMIEDEBERG'S ARCH. PHARMACOL., Bd. 362, 2000, Seiten 116-121,

## Beschreibung

Die Erfindung betrifft die Verwendung von (+)-Tramadol bzw. O-Demethyltramadol, dabei insbesondere (+)-O-Demethyltramadol, O-desmethyl-N-mono-desmethyl-tramadol, dabei insbesondere (+)-O-desmethy)-N-mono-desmethyl-tramadol, als freie Basen und/oder in Form physiologisch verträglicher Salze zur Herstellung eines Arzneimittels zur Behandlung von vermehrtem Harndrang bzw. Harninkontinenz.

Harninkontinenz ist der unwillkürliche Harnabgang. Dieser tritt unkontrolliert auf, wenn der Druck innerhalb der Harnblase den Druck übersteigt, der zum Schließen des Harnleiters notwendig ist. Ursachen können zum einen ein erhöhter interner Blasendruck (z. B. durch Detrusorinstabilität) mit der Folge der Dranginkontinenz und zum anderen ein erniedrigter Sphinkterdruck (z. B. nach Geburt oder chirurgischen Eingriffen) mit der Folge der Streßinkontinenz sein. Der Detrusor ist die grob gebündelte mehrschichtige Blasenwandmuskulatur, deren Kontraktion zur Harnentleerung führt, der Sphinkter der Schließmuskel der Harnröhre. Es treten Mischformen dieser Inkontinenzarten sowie die sogenannte Überflußinkontinenz (z. B. bei benigner Prostatahyperplasie) oder Reflexinkontinenz (z. B. nach Rückenmarksschädigungen) auf. Näheres dazu findet sich bei Chutka, D. S. und Takahashi, P. Y., 1998, Drugs 560: 587-595.

Harndrang ist der auf Harnentleerung (Miktion) abzielende Zustand vermehrter Blasenmuskelspannung bei Annäherung an die Blasenkapazität (bzw. bei deren Überschreitung). Dabei wirkt diese Anspannung als Miktionsreiz. Unter einem vermehrten Harndrang versteht man dabei insbesondere das Auftreten vorzeitigen oder gehäuften manchmal sogar schmerzhaften Harndrangs bis hin zum sog. Harnzwang. Das führt in der Folge zu einer deutlich häufigeren Miktion. Ursachen können u.a. Harnblasenentzündungen und neurogene Blasenstörungen sowie auch Blasentuberkulose sein. Es sind aber noch nicht alle Ursachen geklärt.

Vermehrter Harndrang wie auch Harninkontinenz werden als extrem unangenehm empfunden und es besteht ein deutlicher Bedarf bei von diesen Indikationen betroffenen Personen, eine möglichst langfristige Verbesserung zu erreichen.

Üblicherweise werden vermehrter Harndrang und insbesondere Harninkontinenz medikamentös mit Substanzen behandelt, die an den Reflexen des unteren Harntraktes beteiligt sind (Wein, A. J., 1998, Urology 51 (Suppl. 21); 43 - 47). Meistens sind dies Medikamente, die eine hemmende Wirkung auf den Detrusormuskel, der für den inneren Blasendruck verantwortlich ist, haben. Diese Medikamente sind z. B. Parasympatholytika wie Oxybutynin, Propiverin oder Tolterodin, trizyklische Antidepressiva wie Imipramin oder Muskelrelaxantien wie Flavoxat. Andere Medikamente, die insbesondere den Widerstand der Harnröhre oder des Blasenhalses erhöhen, zeigen Affinitäten zu α-Adrenorezeptoren wie Ephedrin, zu β-Adrenorezeptoren wie Clenbutarol oder sind Hormone wie Östradiol. Auch bestimmte Opioide, Diarylmethylpiperazine und -piperidine, sind für diese Indikation in der WO 93/15062 beschrieben. Weiterhin beschreibt die WO 98/52929 die Verwendung selektiver delta opioid Agonisten aus der Klasse der Piperazinyl-benzyl-tetrazole für urogenitale Störungen wie Harninkontinenz.

Die WO 98/46216 zeigte erstmals, daß in den Indikationen des vermehrten Harndrangs und der Harninkontinenz auch Tramadol eingesetzt werden kann. Tramadol - (1RS,2RS)-2-dimethylaminomethyl-1-(3-methoxyphenyl)-cyclohexanol - ist ein Razemat und ein bekanntes zentral wirksames Analgetikum, das eine starke Schmerzhemmung ohne die für Opioide bekannten Nebenwirkungen hervorruft (J. Pharmacol. Exptl. Ther. 267, 331 (1993)).

Die WO 98/40053 offenbart, bei Wirkstoffen, die üblicherweise als Racemat eingesetzt werden wie beispielsweise Tramadol, zur Vermeidung bestimmter Nebenwirkungen stattdessen ein Enantiomer im Überschuß oder sogar ausschließlich anzuwenden. In der EP-A-870 499 wird die orale Anwendung des Tramadol-Metaboliten (+)-O-Demethyltramadol als Schmerzmittel beschrieben. Auch EP0693475 offenbart die Beteiligung der Metaboliten an der analgetischen Wirksamkeit von Tramadol.

Bei den hier in Frage kommenden Indikationen ist aber zu beachten, daß es sich im allgemeinen um sehr langfristige medikamentöse Anwendungen handelt und sich die Betroffenen im Gegensatz zu vielen Situationen, in denen Analgetika eingesetzt werden, einer sehr unangenehmen, aber nicht unaushaltbaren Situation gegenüber sehen. Daher ist hier - noch mehr als bei Analgetika - darauf zu achten, Nebenwirkungen zu vermeiden, will der Betroffene nicht ein Übel gegen das andere tauschen.

Auch die Anwendung von Tramadol ist, auch wenn Tramadol erheblich weniger Nebenwirkungen zeigt als Opioide, in Abhängigkeit von der Dosis mit einigen, zum Teil unangenehmen Nebenwirkungen verbunden. Außerdem sind bei einer dauerhaften Harninkontinenzbehandlung auch analgetische Wirkungen weitgehend unerwünscht. Entsprechend hat die Verwendung des Razemates Tramadol in dieser Indikation Nachteile, denn, auch wenn das Razemat bereits bei niedrigeren Dosierungen Wirkung auf die Blasenfunktion zeigt, als für analgetische Wirkungen benötigt werden, können therapeutische Dosierungen - insbesondere bei bestimmten Patientenkreisen - bereits unerwünschte Nebenwirkungen zeigen.

Aufgabe der vorliegenden Erfindung war es daher, Stoffe aufzufinden, die zur Behandlung von vermehrtem Harndrang bzw. Harninkontinenz hilfreich sind und bevorzugt gleichzeitig geringere Neben- und analgetischen Wirkungen zeigen als aus dem Stand der Technik bekannt.

Überraschenderweise wurde nun gefunden, daß (+)-Tramadol eine hervorragende Wirkung auf die Blasenfunktion besitzt und demzufolge gut zur Behandlung entsprechender Erkrankungen geeignet ist und dies bereits in erheblich niedrigeren Dosierungen als das Razemat.

Dementsprechend ist Erfindungsgegenstand die Verwendung von (+)-Tramadol, wobei unter den Enantiomeren (+)-Tramadol und (-)-Tramadol ausschließlich das (+)-Tramadol verwendet wird,als freie Base und/oder in Form physiologisch verträglicher Salze zur Herstellung eines Arzneimittels zur Behandlung von vermehrtem Harndrang bzw. Harninkontinenz.

Tramadol ist ein Razemat und besteht aus gleichen Mengen an (+)- und (-)-Enantiomeren. Dabei ist aus der Analgesie bekannt, daß die Enantiomere von Tramadol ein vom Razemat abweichendes pharmakologisches Profil besitzen. Das (+)-Enantiomere zeichnet sich durch eine opiatartige analgetische Wirkung aus, die im Vergleich zu Tramadol verstärkt ist, während beim (-)-Enantiomeren eine deutliche Hemmung der Noradrenalin-Wiederaufnahme beobachtet wird. Dabei wurde für (+)- und (-)-Tramadol nachgewiesen, daß sich diese in Abhängigkeit vom Modell in ihrer Wirkung gegenseitig verstärken (Raffa, R. et. al., 1993, J. Pharmacol. Exptl. Ther. 267:331). Dabei liegt es nahe anzunehmen, daß die potente analgetische Wirkung von Tramadol auf dieser sich bedingenden Wirkverstärkung beruht.

Völlig abweichend von diesen Erfahrungen aus dem Bereich der Analgesie wurde bei Untersuchungen der separaten Enantiomere in ihrer Wirkung auf die Blasenfunktion ein überraschend anderes Bild gefunden. (+)-Tramadol war nicht nur deutlich wirksamer als das Razemat sondern sogar erheblich wirksamer als die doppelte Dosis der eingesetzten Racematmischung aus (+)- und (-)-Tramadol. Daraus ist aber zu schliessen, daß (+)-Tramadol nicht nur der eigentlich wirksame Stoff ist, sondern daß (-)-Tramadol nicht nur unwirksam zu sein, sondern im Gegensatz zur Analgesie im Razemat die Wirkung auf die Blasenfunktion von (+)-Tramadol sogar zu hemmen scheint.

So hat die Verwendung von (+)-Tramadol gegenüber dem Stand der Technik, der Verwendung von Tramadol als Razemat (WO 98/46216), klare Vorteile, da erheblich niedriger dosiert werden kann, deutlich weniger als 50 % der für Tramadol nötigen Dosierung. Entsprechend niedriger sind die Nebenwirkungen, da auch (-)-Tramadol zu diesen, insbesondere auch den analgetischen Wirkungen, beiträgt. Möglichkeiten zur Herstellung von (+)-Tramadol sind in Arzneim.-Forsch./Drug Res. 28 (I), 114 (1978) und insbesondere bevorzugt in der DE 196 01 745 C1 beschrieben.

Geeignete Salze im Sinne dieser Erfindung und in jeder der beanspruchten Verwendungen sind Salze des jeweiligen Wirkstoffes mit anorganischen bzw. organischen Säuren und/oder einem Zuckeraustauschstoff wie Saccharin, Cyclamat oder Acesulfam. Besonders bevorzugt ist jedoch das Hydrochlorid.

Ein weiterer Gegenstand der vorliegenden Erfindung ist die Verwendung von O-Demethyltramadol und/oder seiner Enantiomeren, Diastereomeren, Basen oder Salze physiologisch verträglicher Säuren zur Herstellung eines Arzneimittels zur Behandlung von vermehrtem Harndrang bzw. Harninkontinenz. Bevorzugt ist dabei insbesondere die Verwendung von (+)-O-Demethyltramadol als freie Base und/oder in Form physiologisch verträglicher Salze. Tramadol bildet in vivo den Metaboliten O-Demethyltramadol, der gleichfalls als Enantiomerengemisch vorliegt. In Hinsicht auf analgetische Wirkungen haben Untersuchungen ergeben, daß sowohl die beiden Tramadol-Enantiomere als auch die beiden Enantiomere der Tramadol metabolite an der analgetischen Wirkung beteiligt sind (J. Pharmacol. Exptl. Ther. 260, 275 (1992); Arzneim. Forschung 38, 877 (1988)).

Überraschenderweise hatte auch das Razemat O-Demethyltramadol bereits bei niedrigen Konzentrationen eine deutliche Wirkung auf die Blasenfunktion. Bei genauerer Untersuchung der Enantiomere zeigte es sich, daß (+)-O-Demethyltramadol für die gesamte Wirkung auf die Blasenfunktion verantwortlich zu sein scheint. Die Herstellung von O-Demethyltramadol als Razemat oder in Form der Enantiomeren ist aus EP 534 628 und WO 93/04675 bekannt. Die Herstellung des Enantiomers (+)-O-Demethyltramadol erfolgt bevorzugt nach dem in DE 196 01 744 C2 beschriebenen Verfahren.

Bei der Verwendung von (+)-O-Demethyltramadol ist es nicht notwendig, aber bevorzugt, ausschließlich das (+)-O-Demethyltramadol Enantiomere zu verwenden. Ein gegenüber dem (+)-O-Demethyltramadol geringerer Anteil an (-)-O-Demethyltramadol ist aber akzeptabel und darf bei der erfindungsgemässen Verwendung enthalten sein.

Ein weiterer Gegenstand der vorliegenden Erfindung ist die Verwendung von O-desmethyl-N-mono-desmethyl-tramadol und/oder seiner Enantiomeren; insbesondere Mischungen seiner Enantiomere oder eines einzelnen Enantiomers; Diastereomeren, Basen oder Salze physiologisch verträglicher Säuren zur Herstellung eines Arzneimittels zur Behandlung von vermehrtem Harndrang bzw. Harninkontinenz. Bevorzugt ist dabei insbesondere die Verwendung von (+)-O-desmethyl-N-mono-desmethyltramadol_als freie Base und/oder in Form physiologisch verträglicher Salze.

O-desmethyl-N-mono-desmethyl-tramadol (an einigen Stellen des folgenden Textes und in der Literatur als M5 bezeichnet) ist als einer der Invivo-Metabolite von Tramadol (1RS,2RS)-2[(dimethylamino)methyl]-1-(3-methoxyphenyl)-cyclohexanol bekannt ( Lintz et al., Arzneim.-Forsch./Drug Res. 31(11), 1932-1943, 1981). M5 weist nur eine geringe Durchdringung der Bluthirnschranke auf, da zentralnervöse Wirkungen - z.B. analgetische - bei i.v.-Gabe im Gegensatz zur i.c.v.-Gabe deutlich schwächer ausgeprägt sind. Überraschenderweise hatte auch das Razemat O-Demethyltramadol bereits bei niedrigen Konzentrationen eine deutliche Wirkung auf die Blasenfunktion. Bei genauerer Untersuchung der Enantiomere zeigte es sich, daß (+)-O-desmethyl-N-mono-desmethyltramadol für die gesamte Wirkung auf die Blasenfunktion verantwortlich zu sein scheint.

Bei der Verwendung von (+)-O-desmethyl-N-mono-desmethyl-tramadol ist es nicht notwendig, aber bevorzugt, ausschließlich das (+)-O-desmethyl-N-mono-desmethyl-tramadol Enantiomere zu verwenden. Ein gegenüber dem (+)-O-desmethyl-N-mono-desmethyl-tramadol geringerer Anteil an (-)-O-desmethyl-N-mono-desmethyl-tramadol ist aber akzeptabel und darf bei der erfindungsgemässen Verwendung enthalten sein.

Auch wenn die erfindungsgemässen Verwendungen lediglich geringe Nebenwirkungen zeigen, kann es beispielsweise zur Vermeidung von bestimmten Formen der Abhängigkeit von Vorteil sein, neben (+)-Tramadol, O-Demethyltramadol oder (+)-O-Demethyltramadol, O-desmethyl-N-mono-desmethyl-tramadol oder (+)-O-desmethyl-N-mono-desmethyl-tramadol auch Morphinantagonisten, insbesondere Naloxon, Naltrexon und/oder Levallorphan, zu verwenden.

Geeignete Salze im Sinne dieser Erfindung und in jeder der beanspruchten Verwendungen sind Salze des jeweiligen Wirkstoffes mit anorganischen bzw. organischen Säuren und/oder einem Zuckeraustauschstoff wie Saccharin, Cyclamat oder Acesulfam. Besonders bevorzugt ist jedoch das Hydrochlorid.

Die folgenden Beispiele sollen die Erfindung erläutern.

### Beispiele

### Beispiel 1: Testsystem Cystometrie an der wachen naiven Ratte

Es wurden cystometrische Untersuchungen an naiven, weiblichen Sprague-Dawley-Ratten nach der Methode von Ishizuka et. al. ((1997), Naunyn-Schmiedeberg's Arch. Pharmacol. 355: 787 - 793) durchgeführt. Drei Tage nach Implantation von Blasen- und venösen Kathetern wurden die Tiere im wachen Zustand, frei beweglich untersucht. Der Blasenkatheter wurde an einem Druckaufnehmer und eine Injektionspumpe angeschlossen. Die Tiere wurden in Stoffwechselkäfige gesetzt, die die Messung des Harnvolumens ermöglichten. Physiologische Kochsalzlösung wurde in die entleerte Blase infundiert (10 ml/Std.) und Blasendruck und Miktionsvolumen kontinuierlich aufgezeichnet. Nach einer Stabilisierungsphase wurde eine 20minütige Phase aufgezeichnet, die durch normale, reproduzierbare Miktionszyklen gekennzeichnet war. Im einzelnen wurden die folgenden Parameter bestimmt:

Miktionsdruck (micturition pressure MP, maximaler Druck während der Miktion), Basaldruck (basal pressure BP, niedrigster Druck während der Füllungsphase), Schwellendruck (threshold pressure TP, Blasendruck unmittelbar vor Miktion), Blasenkapazität (bladder capacity BC, Restvolumen nach vorhergehender Miktion plus Volumen der infudierten Lösung während der Füllungsphase), Miktionsvolumen (micturition volume MV, Volumen des abgesetzten Harns) und Restvolumen (Residual volume RV, Blasenkapazität abzüglich des Miktionsvolumens). Besonderes Augenmerk ist dabei auf den Schwellendruck (TP) zu richten, da eine Erhöhung eine wichtige therapeutische Wirkung bei einer der erfindungsgemässen Indikationen anzeigt.

### Beispiel 2: Vergleich der Referenzsubstanzen razemisches Tramadol und (-) Tramadol mit der Testsubstanz (+) -Tramadol

Nach der Aufzeichnung von drei reproduzierbaren Miktionszyklen als Vorwert, wurden die Testsubstanzen (Tramadol, 10 mg/kg i. v.; (+)-Tramadol, 5 mg/kg i. v.; (-)-Tramadol, 5 mg/kg i. v.; Vehikel = 0,9 % NaCl) appliziert und die Wirkung auf die cystometrischen Parameter 90 bis 120 Minuten aufgezeichnet. Im Wirkmaximum wurde der Mittelwert von 3 Miktionszyklen bestimmt und als prozentuale Veränderung gegenüber dem Vorwert dargestellt (Tabelle 1).

**Tabelle 1: Beeinflussung der cystometrischen Parameter durch Tramadol und seine Enantiomere Angegeben sind Durchschnittswerte mit Standardabweichung vor (v) und nach Anwendung (h) der Testsubstanz sowie die Differenz (Diff.), die Veränderung im Vergleich zum Vorwert [%]; n entspricht der Anzahl der Versuche; Signifikanz (Student T-Test): * p < 0.05; ** p < 0.01; *** p < 0.001.**

| | **micturition pressure** [cm H₂O] | **threshold pressure** [cm H₂O] | **basal pressure** [cm H₂O] | **bladder capacity** [ml] | **micturition volume** [ml] | **residual volume** [ml] |
|---|---|---|---|---|---|---|
| Tramadol | v: 74 ± 5 | v: 15.01 ± 1.32 | v: 10.21 ± 1.03 | v: 0.95 ± 0.06 | v: 0.85 ± 0.05 | v: 0.10 ± 0.02 |
| 10.0 mg/kg i.v. | h: 66 ± 5 | h: 25.50 ± 2.40*** | h: 14.31 ± 1.89* | h:0.78 ± 0.08 | h: 1.13 ± 0.08** | h: 0.05 ± 0.005 |
| (n=9) | Diff.:-11% | Diff.: + 70% | Diff.: + 40 % | Diff.: - 18 % | Diff.: + 33 % | Diff.: - 50% |
| (+)-Tramadol | v: 60 ± 4 | v: 8.0 ± 1.10 | v: 4.23 ± 0.35 | v: 0.92 ± 0.08 | v: 0.81 ± 0.07 | v: 0.11 ± 0.02 |
| 5.0 mg/kg i.v. | h: 51 ± 7 | h: 19.20 ± 3.02** | h: 6.46 ± 1.13* | h: 0.90 ± 0.09 | h: 1.18 ± 0.12*** | h: 0.006 ± 0.003** |
| (n=9) | Diff.: 15% | Diff.: + 140% | Diff.: + 53 % | Diff.: - 2% | Diff.: + 46 % | Ditt.: - 95 % |
| (-)-Tramadol | v: 69 ± 4 | v: 7.32 ± 2.21 | v: 4.12 ± 0.40 | v: 1.19 ± 0.13 | v: 1.07 ± 0.13 | v: 0.12 ± 0.01 |
| 5.0 mg/kg i.v. | h: 59 ± 3* | h: 7.10 ± 0.44 | h: 4.20 ± 0.30 | h: 1.10 ± 0.09* | h: 1.01 ± 0.09 | h: 0.10 ± 0.02 |
| (n=10) | Diff.: - 15% | Diff.: - 3% | Diff.: + 2 % | Diff.: - 8 % | Diff.: - 6 % | Diff.: - 17 % |

Tramadol (10 mg/kg i. v) zeigte hier den erwarteten Effekt. Es war ein deutlicher Anstieg des Schwellendrucks festzustellen. Dies spricht für eine positive Wirkung des Tramadol Razemats auf die Blasenfunktion, wie bereits aus der WO 98/46216 bekannt war.. Bei der Untersuchung der beiden Enantiomere (jeweils 5 mg/kg i. v.) stellte sich hingegen heraus, daß nur das (+)-Enantiomer nicht aber das (-)-Enantiomer einen Effekt auf die Blasenparameter ausübt. Dabei übersteigt der Effekt von 5 mg/kg Tramadol den Effekt von 10 mg/kg razemischen Tramadol deutlich. Im Bereich des Schwellendrucks ist sogar die doppelte Wirkung festzustellen. Daraus läßt sich schließen, daß (+)-Tramadol Träger der Aktivität des Razemats ist und daß (-)- Tramadol im Racemat wahrscheinlich nicht nur unwirksam ist, sondern vermutlich sogar einen hemmenden Effekt hat.

Daher ist hier nachgewiesen, daß (+)-Tramadol eine deutlich bessere Wirkung auf die Blasenfunktion besitzt als das razemische Tramadol.

### Beispiel 3: Vergleich der Referenzsubstanz razemisches Tramadol mit den Testsubstanzen (+)-Tramadol und (+)-O-Desmethyltramadol

In einem analogen Versuch gemäß Beispiel 1 wurden - nach der Aufzeichnung von drei reproduzierbaren Miktionszyklen als Vorwert - razemisches Tramadol ((r)-Tram) (1,0 und 5,0 mg/kg), (+)-Tramadol ((+)-Tram) (0,1, 0,3, und 0,5 mg/kg) und (+)-O-Desmethyltramadol ((+)-M1) (0,1 und 0,5 mg/kg) im Vehikel 0,9 % NaCl i.v. appliziert und die Wirkung auf die cystometrischen Parameter 90 bis 120 Minuten aufgezeichnet. Im Wirkmaximum wurde der Mittelwert von 3 Miktionszyklen bestimmt und als prozentuale Veränderung gegenüber dem Vorwert dargestellt (Tabelle 2). Zum Vergleich sind Daten aus Tab. 1 erneut aufgeführt.

Zusätzlich zum vorherigen Versuch wurde hier das "inter-contraction interval", das Zeitintervall zwischen den Miktionen, in Minuten gemessen. Das "inter-contraction interval" ist auch ein wichtiger Parameter zur Messung der physiologischen Wirksamkeit eines Stoffes in der Behandlung der Haminkontinenz.

**Tabelle 2: Beeinflussung der cystometrischen Parameter durch Tramadol und seine Enantiomere sowie Metabolite. Angegeben sind Durchschnittswerte mit Standardabweichung vor (v) und nach Anwendung (h) der Testsubstanz sowie die Differenz (Diff.), die Veränderung im Vergleich zum Vorwert [%]; n entspricht der Anzahl der Versuche; Signifikanz (Student T-Test): * p < 0.05; ** p < 0.01; *** p < 0.001. Mit markierte Werte sind Wiederholungen aus Beispiel 2.**

| | micturition pressure [cm H₂O] | threshold pressure [cm H₂O] | basal pressure [cm H₂O] | bladder capacity [ml] | micturition volume [ml] | residual volume [ml] | Inter-contraction interval [min] |
|---|---|---|---|---|---|---|---|
| (r)-Tram | v: 94 ± 7 | v: 14.0 ± 2.04 | v: 9.02 ± 1.12 | v: 0.84 ± 0.11 | v: 0.75 ± 0.09 | v: 0.09 ± 0.02 | |
| 1,0 mg/kg i.v. | h: 85 ± 6* | h: 21.03 ± 3.10*** | h: 11.34 ± 1.54* | h: 0.82 ± 0.09 | h: 0.95 ± 0.08* | h: 0.01 ± 0.005 | |
| (n = 6) | Diff.: - 10 % | Diff.: + 50 % | Diff.: + 26 % | Diff.: - 2 % | Diff.: + 27 % | Diff.: - 89 % | |
| (r)-Tram | v: 103 ± 10 | v: 12.45 ± 1.41 | v: 9.21 ± 1.35 | v: 0.87 ± 0.07 | v: 0.76 ± 0.06 | v: 0.11 ± 0.02 | |
| 5,0 mg/kg i.v. | h: 75 ± 10*** | h: 17.05 ± 2.33* | h: 9.30 ± 2.10 | h: 0.92 ± 0.08 | h: 1.08 ± 0.09*** | h: 0.03 ± 0.01* | |
| (n = 10) | Diff.: - 27 % | Diff.: + 37 % | Diff.: + 1 % | Diff.: + 6 % | Diff.: + 42 % | Diff.: - 73 % | Diff.: - 11 % |
| (r)-Tram | v: 74 ±5 | v: 15.01 ±1.32 | v: 10.21 ±1.03 | v: 0.95 ± 0.06 | v: 0.85 ± 0.05 | v: 0.10 ± 0.02 | |
| 10,0 mg/kg i.v. | h: 66 ± 5 | h: 25.50 ± 2.40*** | h: 14.31 ± 1.89* | h:0.78 ± 0.08 | h: 1.13 ± 0.08** | h: 0.05 ± 0.005 | |
| (n = 9) | Diff.: -11% | Diff.: + 70% | Diff.: + 40 % | Diff.: - 18 % | Diff.: + 33 % | Diff.: - 50% | Diff.: - 40 % |
| (+)-Tram | v: 69± 11 | v: 7.87 ± 1.51 | v: 4.01 ± 0.57 | v: 0.91 ± 0.11 | v: 0.75 ± 0.12 | v: 0.16 ± 0.01 | v: 5.36 ± 0.63 |
| 0,1 mg/kg i.v. | h: 55 ± 8* | h: 11.63 ± 1.05* | h: 3.85 ± 0.46 | h: 1.07 ± 0.13* | h: 0.80 ± 0.09 | h: 0.23 ± 0.07 | h: 7.13 ± 0.85* |
| (n = 5) | Diff.: - 20 % | Diff.: + 48 % | Diff.: - 4 % | Diff.: + 18 % | Diff.: + 7 % | Diff.: + 44 % | Diff.: + 33 % |
| (+)-Tram | v: 71 ± 4 | v: 8.21 ± 0.42 | v: 4.50 ± 0.29 | v: 0.99 ± 0.08 | v: 0.86 ± 0.07 | v: 0.15 ± 0.01 | v: 6.06 ± 0.49 |
| 0,3 mg/kg i.v. | h: 60 ± 5" | h: 16.01 ± 63** | h: 4.95 ± 0.19 | h: 1 .38 ± 0.14** | h: 0.97 ± 0.13 | h: 0.40 ± 0.04** | h: 7.21 ± 0.72* |
| (n = 8) | Diff.: - 15 % | Diff.: + 95 % | Dift.: + 10 % | Diff.: + 39 % | Diff.: + 13 % | Diff.: + 167 % | Diff.: + 19 % |
| (+)-Tram | v: 73 ± 12 | v: 7.85 ± 1.04 | v: 4.16 ± 0.62 | v: 1.21 ± 0.06 | v: 1.07 ± 0.06 | v: 0.14 ± 0.03 | |
| 0,5 mg/kg i.v. | h: 57 ± 10* | h: 15.0 ± 2.58* | h: 4.70 ± 1.14 | h: 1.43 ± 0.11* | h: 1.31 ± 0.12* | h: 0.12 ± 0.002 | |
| (n = 5) | Diff.: - 22 % | Diff.: + 91 % | Diff.: + 13 % | Diff.: + 18 % | Diff.: + 22 % | Diff.: - 14 % | Diff.: + 21 % |
| (+)-Tram | v: 60 ± 4 | v: 8.0 ± 1.10 | v: 4.23 ± 0.35 | v: 0.92 ± 0.08 | v: 0.81 ± 0.07 | v: 0.11 ± 0.02 | |
| 5,0 mg/kg i.v. | h: 51 ± 7 | h:19.20 ± 3.02** | h: 6.46 ± 1.13* | h: 0.90 ± 0.09 | h: 1.18 ± 0.12*** | h:0.006 ± 0.003** | |
| (n = 10) | Diff.: - 15% | Diff.: + 140% | Diff.: + 53 % | Diff.: - 2% | Diff.: + 46 % | Diff.: - 95 % | Diff.: + 7 % |
| (+)-M1 | v: 90 ± 12 | v: 6.12 ± 0.51 | v: 3.62 ± 0.24 | v: 0.75 ± 0.08 | v: 0.64 ± 0.10 | v: 0.11 ± 0.02 | 4.20 ± 0.55 |
| 0,1 mg/kg i.v. | h: 70 ± 11 | h: 8.07 ± 0.97 | h: 3.87 ± 0.43 | h: 0.98 ± 0.06** | h: 0.95 ± 0.09* | h: 0.03 ± 0.02 | 5.51 ± 0.52 |
| (n = 5) | Diff.:-22% | Diff.: + 32 % | Diff.: + 7 % | Diff.: + 31 % | Diff.: + 48 % | Diff.: - 73 % | Diff.: + 31 % |
| (+)-M1 | v: 94 ± 10 | v: 7.44 ± 1.10 | v: 3.91 ± 0.31 | v: 0.94 ± 0.08 | v: 0.85 ± 0.08 | v: 0.09 ± 0.02 | 5.23 ± 0.45 |
| 0,5 mg/kg i.v. | h: 66 ± 10** | h: 15.34 ± 1.16*** | h: 4.90 ± 0.55* | h: 1.35 ± 0.11 ** | h: 1.28 ± 0.09*** | h: 0.06 ± 0.02 | 7.63 ± 0.60** |
| (n =8) | Diff.: - 30 % | Diff.: + 106 % | Diff.: + 25 % | Diff.: + 44 % | Diff.: + 51 % | Diff.: - 33 % | Diff.: + 46 % |

Insgesamt ist bei der Beurteilung der Wirksamkeit in der Harninkontinenz besonderer Wert auf den Schwellendruck (Threshold presure (TP)), die Blasenkapazität (Bladder capacity (BC)) und das Intervall zwischen den Kontraktionen (inter-contraction interval (ICI)) zu legen. (r)-Tram war erst in erheblich höheren Dosen wirksam als (+)-Tram und zeigte bei der effektivsten Dosis (10 mg/kg) eine deutliche Verkürzug des ICI. Gerade letzteres ist aber eine bei der Behandlung von Harninkontinenz äußerst ungünstige Nebenwirkung. (+)-Tram ist dem razemischen Tramadol insgesamt deutlich überlegen. So erzielt z.B. (+)-Tram auch eine deutliche Steuigerung der Blasenkapazität. Noch besser allerdings erscheint das (+)-O-desmethyl-Tramadol sowohl bei 0,1 als auch insbesondere bei 0,5 mg/kg i. v.. Es war insbesondere ein deutlicher Anstieg des Schwellendrucks festzustellen. Auch die Blasenkapazität wurde erhöht und das Intervall zwischen den Miktionen verlängert. Damit ist eine positive Wirkung des O-desmethyl-Tramadols, insbesondere des (+)-O-desmethyl-Tramadols auf die Blasenfunktion nachgewiesen, die razemisches Tramadol deutlich und in entscheidenden Parametern sogar die des (+)-Tramadols zu übertreffen scheint.

### Beispiel 4: Wirksamkeit von O-desmethyl-N-mono-desmethyltramadol, insbesondere (+)-O-desmethyl-N-mono-desmethyl-tramadol

In einem analogen Versuch gemäß Beispiel 1 wurden - nach der Aufzeichnung von drei reproduzierbaren Miktionszyklen als Vorwert - (+)-O-desmethyl-N-mono-desmethyl-tramadol ((+)-M5) (10 mg/kg) im Vehikel 0,9 % NaCl i.v. appliziert und die Wirkung auf die cystometrischen Parameter 90 bis 120 Minuten aufgezeichnet. Im Wirkmaximum wurde der Mittelwert von 3 Miktionszyklen bestimmt und als prozentuale Veränderung gegenüber dem Vorwert dargestellt (Tabelle 3). Da aber für M5, bzw. (+)-M5 nicht bekannt ist, ob es die Blut/Himschranke überwinden kann, und die Wirkung möglicherweise zentral erfolgt, wurden 10 µg/kg intrathekal (it) appliziert (Tabelle 3). Zusätzlich zum Beispiel 1 wurde hier das "inter-contraction interval", das Zeitintervall zwischen den Miktionen, in Minuten gemessen.

**Tabelle 3: Beeinflussung der cystometrischen Parameter durch (+)-M5. Angegeben sind Durchschnittswerte mit Standardabweichung vor (v) und nach Anwendung (h) der Testsubstanz sowie die Differenz (Diff.), die Veränderung im Vergleich zum Vorwert [%]; n entspricht der Anzahl der Versuche; Signifikanz (Student T-Test): *p < 0.05; ** p < 0.01; *** p < 0.001.**

| | micturition pressure [cm H₂O] | threshold pressure [cm H₂O] | basal pressure [cm H₂O] | bladder capacity [ml] | micturition volume [ml] | residual volume [ml] | Inter-contraction interval [min] |
|---|---|---|---|---|---|---|---|
| (+)-M5 | v: 53 ± 4 | v: 7.13 ± 0.47 | v: 3.78 ± 0.26 | v: 0.88 ± 0.09 | v: 0.79 ± 0.09 | v: 0.08 ± 0.01 | v:4.91 ± 0.53 |
| 10,0 mg/kg i.v. | h: 41 ± 3** | h: 12.08 ± 1.47* | h: 3.95 ± 0.35 | h: 0.72 ± 0.08* | h: 0.86 ± 0.10 | h: 0.01 ± 0.002** | h:4.30 ± 0.47* |
| (n = 6) | Diff.: - 23 % | Diff.: + 69 % | Diff.: + 4 % | Diff.: - 18 % | Diff.: + 9 % | Diff.: - 88 % | Diff.: - 12 % |
| (+)-M5 | v: 80 ± 9 | v: 7.0 ± 0.93 | v: 4.68 ± 0.74 | v: 0.66 ± 0.05 | v: 0.53 ± 0.06 | v: 0.13 ± 0.01 | v:3.79 ± 0.35 |
| 10,0 µg/kg i.t. | h: 43 ± 6*** | h: 10.25 ± 1.01* | h: 4.25 ± 0.33 | h: 1.06 ± 0.06** | h: 0.72 ± 0.04* | h: 0.34 ± 0.04* | h:5.22 ± 0.27** |
| (n = 6) | Diff.: - 46 % | Diff.: + 46 % | Diff.: - 9 % | Diff.: + 61 % | Diff.: + 36 % | Diff.: + 162 % | Diff.: + 38 % |

Auch (+)-O-desmethyl-N-mono-desmethyl-tramadol ist wirksam und bei 10,0 mg/kg i.v. dem Tramadol vergleichbar. Es war ein deutlicher Anstieg des Schwellendrucks festzustellen, wobei sich bei (+)-M5 das Intervall zwischen den Miktionen nicht verkürzte. Intrathekal ist (+)-M5 ebenfalls wirksam und bewirkt neben einer Erhöhung des Schwellendrucks deutliche Steigerungen der Blasenkapazität und des Intervalls zwischen den Miktionen. Damit ist eine positive Wirkung des O-desmethyl-N-mono-desmethyl-tramadol, insbesondere des (+)-O-desmethyl-N-mono-desmethyl-tramadol auf die Blasenfunktion nachgewiesen.

## Patentansprüche

1. Verwendung von (+)-Tramadol, wobei unter den Enantiomeren (+)-Tramadol und (-)-Tramadol ausschließlich das (+)-Tramadol verwendet wird, als freie Base und/oder in Form physiologisch verträglicher Salze zur Herstellung eines Arzneimittels zur Behandlung von vermehrtem Harndrang bzw. Harninkontinenz.

2. Verwendung von O-Demethyltramadol und/oder seiner Enantiomeren, Diastereomeren, Basen oder Salze physiologisch verträglicher Säuren zur Herstellung eines Arzneimittels zur Behandlung von vermehrtem Harndrang bzw. Harninkontinenz.

3. Verwendung von gemäß Anspruch 2 (+)-O-Demethyltramadol als freie Base und/oder in Form physiologisch verträglicher Salze zur Herstellung eines Arzneimittels zur Behandlung von vermehrtem Harndrang bzw. Harninkontinenz.

4. Verwendung von O-desmethyl-N-mono-desmethyl-tramadol und/oder seiner Enantiomeren, Diastereomeren, Basen oder Salze physiologisch verträglicher Säuren zur Herstellung eines Arzneimittels zur Behandlung von vermehrtem Harndrang bzw. Harninkontinenz.

5. Verwendung gemäß Anspruch 4 von (+)-O-desmethyl-N-mono-desmethyl-tramadol als freie Base und/oder in Form physiologisch verträglicher Salze zur Herstellung eines Arzneimittels zur Behandlung von vermehrtem Harndrang bzw. Harninkontinenz.

6. Verwendung gemäß einem der Ansprüche 1 bis 5 **dadurch gekennzeichnet, daß** das Salz ein Hydrochlorid ist.

## Claims

1. Use of (+)-tramadol, wherein, of the enantiomers (+)-tramadol and (-)-tramadol, solely (+)-tramadol is used, as a free base and/or in the form of physiologically acceptable salts for the production of a pharmaceutical preparation for the treatment of increased urinary urgency or urinary incontinence.

2. Use of O-demethyltramadol and/or its enantiomers, diastereomers, bases or salts of physiologically acceptable acids for the production of a pharmaceutical preparation for the treatment of increased urinary urgency or urinary incontinence.

3. Use according to claim 2 of (+)-O-demethyltramadol as a free base and/or in the form of physiologically acceptable salts for the production of a pharmaceutical preparation for the treatment of increased urinary urgency or urinary incontinence.

4. Use of O-desmethyl-N-mono-desmethyltramadol and/or its enantiomers, diastereomers, bases or salts of physiologically acceptable acids for the production of a pharmaceutical preparation for the treatment of increased urinary urgency or urinary incontinence.

5. Use according to claim 4 of (+)-O-desmethyl-N-mono-desmethyltramadol as free base and/or in the form of physiologically acceptable salts for the production of a pharmaceutical preparation for the treatment of increased urinary urgency or urinary incontinence.

6. Use according to any one of claims 1 to 5, **characterised in that** the salt is a hydrochloride.

## Revendications

1. Utilisation du (+)-tramadol, à savoir exclusivement du (+)-tramadol entre les énantiomères (+)-tramadol et (-)-tramadol, sous forme de base libre et/ou sous forme de sels physiologiquement acceptables pour la préparation d'un médicament destiné au traitement de la répétition du besoin impérieux d'uriner ou de l'incontinence urinaire.

2. Utilisation du O-déméthyltramadol et/ou de ses énantiomères, diastéréoisomères, bases ou sels d'acides physiologiquement acceptables pour la préparation d'un médicament destiné au traitement de la répétition du besoin impérieux d'uriner ou de l'incontinence urinaire.

3. Utilisation suivant la revendication 2 du (+)-O-déméthyltramadol sous forme de base libre et/ou sous forme de sels physiologiquement acceptables pour la préparation d'un médicament destiné au traitement de la répétition du besoin impérieux d'uriner ou de l'incontinence urinaire.

4. Utilisation du O-desméthyl-N-monodesméthyltramadol et/ou de ses énantiomères, diastéréoisomères, bases ou sels d'acides physiologiquement acceptables pour la préparation d'un médicament destiné au traitement de la répétition du besoin impérieux d'uriner ou de l'incontinence urinaire.

5. Utilisation suivant la revendication 4 du (+)-O-desméthyl-N-monodesméthyltramadol sous forme de base libre et/ou sous forme de sels physiologiquement acceptables pour la préparation d'un médicament destiné au traitement de la répétition du besoin impérieux d'uriner ou de l'incontinence urinaire.

6. Utilisation suivant l'une des revendications 1 à 5, **caractérisée en ce que** le sel est le chlorhydrate.
